# EUROPÄISCHE PATENTANMELDUNG

(11) **EP 1 066 834 A2**
(43) Veröffentlichungstag der Anmeldung: **10.01.2001**
(21) Anmeldenummer: 00114206.6
(22) Anmeldetag: 03.07.2000
(51) Int. Cl.: A61K 35/14, A61K 38/55, A61K 38/00, A61K 31/00, A61K 35/00, A61K 33/00, A61P 7/02, A61P 7/04, A61P 31/00

(54) **Pharmazeutische Zusammensetzungen enthaltend ein phagozytenmodulierendes Agens**

(30) Priorität: 08.07.1999 DE 19940945; 08.08.1999 DE 19936744
(71) Anmelder: Stief, Thomas, Dr., 35415 Pohlheim (DE)
(72) Erfinder: Stief, Thomas, Dr., 35415 Pohlheim (DE)
(74) Vertreter: Ackermann, Joachim, Dr.

(57) **Zusammenfassung**

Beschrieben werden pharmazeutische Zusammensetzungen zur Behandlung oder zur Prophylaxe von thrombotischen, atheroskierotischen, hämorragischen oder infektösen Erkrankungen. Die erfindungsgemäße pharmazeutische Zusammensetzung enthält ein Agens, das die Aktivität von Phagozyten moduliert, insbesondere aktiviert.

## Beschreibung

Die vorliegende Erfindung betrifft pharmazeutische Zusammensetzungen zur Behandlung oder zur Prophylaxe von thrombotischen, atherosklerotischen, hämorrhagischen oder infektösen Erkrankungen.

Hämostase ist das System des Auf- und Abbaus von Thromben. Hämostase besteht aus Gerinnung und Fibrinolyse. Neben der plasmatischen gibt es eine zelluläre Komponente der Hämostase, woran Thrombozyten und Endothelzellen beteiligt sind. Hämostase und Entzündung/Immunantwort sind oftmals gekoppelte Systeme. An der Pathogenese der Atherosklerose/Arteriosklerose ist eine Störung der Hämostase im Sinne einer Atherothrombose beteiligt.

Eine Dysfunktion sowohl der Gerinnung als auch der Fibrinolyse kann lebensgefährlich sein: Gerinnungsüberfunktion und/oder Fibrinolyseunterfunktion können zu Thrombosen fuhren, Gerinnungsunterfunktion und/oder Fibrinolyseüberfunktion zu Blutungen.

Nach dem Stand der Technik sind alle Thrombolytika, die derzeit klinisch verwendet werden, Plasminogen-Aktivatoren, wie Streptokinase, Plasminogen-Streptokinase-Aktivator-Komplex, Urokinase, Pro-Urokinase oder Gewebs-Plasminogen-Aktivator. Diese sind kostenintensiv und führen aufgrund ihrer Unselektivität nicht selten zu lebensbedrohlichen Blutungen.

Aus der DE-A-197 12 565 sind pharmazeutische Zusammensetzungen bekannt, die ein von einer Anregungsstrahlung unabhängiges Sigulett-Sauerstoff und/oder Photonen erzeugendes Agenz oder eine Vorstufe desselben enthalten. Die Verwendung dieser Zusammensetzungen als Antithrombotikum oder als Antivirikum wird beschrieben. Mit der vorbekannten Zusammensetzung soll die Erzeugung von Singulett-Sauerstoff oder Photonen erreicht werden, ohne daß eine Anregungsbestrahlung notwendig ist. Hinweise auf eine Phagozytenmodulation lassen sich aus dieser Schrift nicht ableiten.

Es wurde nun überraschenderweise gefunden, daß Thromben insbesondere in vivo auch zerstört werden können und/oder deren Entstehung insbesondere in vivo verhindert werden kann, indem die Phagozyten des Blutes (im Besonderen die polymorphkernigen neutrophilen Granulozyten (PMN) und die Monozyten, insbesondere die PMN) aktiviert (stimuliert) werden.

Die phagozytäre Thrombolyse ist durch eine typische Thrombushistologie gekennzeichnet: nach PMN-Aktivierung nimmt die Konzentration an Phagozyten (vor allem an PMN) im Gerinnsel mehr als 1000-fach zu. Diese phagozytäre Zerstörung des Thrombus richtet sich spezifisch (selektiv) auf den Thrombus und nicht auf Faktoren des plasmatischen Gerinnungssystems, was schwere Blutungen (z.B. Cerebralblutungen) verursachen kann. Diese Komplikation der herkömmlich in der Klinik verwendeten Thrombolytika ist sehr geffirchtet und trägt deswegen dazu bei, daß die Indikation für die herkömmliche Thrombolyse sehr streng gestellt wird (z.B. möglichst junger Patient, Myokardinfarkt- oder Apoplex-Beginn innerhalb der letzten 4-6 bzw. 3 Stunden).

Die erfindungsgemäße Phagozytenaktivierung ist hier gegenüber der herkömmlichen klinischen Thrombolyse von Vorteil und gestattet den möglicherweise lebensrettenden Einsatz erfindungsgemäßer Antithrombotika und/oder Thrombolytika bei einem wesentlich breiteren Patientenkollektiv und - bedingt durch die geringe Nebenwirkungsrate - bereits durch den erstversorgenden Arzt (und nicht erst Stunden nach Infarkt-, Apoplex-Beginn durch den Stationsarzt der Intensivstation).

Überraschenderweise wirken Phagozytenaktivatoren auch antikoagulant, so daß durch deren Verwendung eine atherothrombotische Erkrankung (Atherosklerose / Arteriosklerose und/oder Thrombose wie bei Myokardinfarkt oder Apoplex) nicht nur therapiert sondern ihr auch vorgebeugt wird.

Andererseits gibt es hämostaseologische Erkrankungen, die mit einer Überfunktion von Phagozyten einhergehen (d.h. phagozytenaktivierungsbedingt sind; wie bestimmte Hämorrhagien, beispielsweise wie bei Zuständen von disseminierter intravasaler Gerinnung (Verbrauchskoagulopathie) oder bei Formen der Cerebralblutung, beispielsweise bei Zuständen nach Subarachnoidalblutung, bei denen es zu einer erhöhten Aktivierung von Phagozyten kommt).

Phagozyten- (insbesondere PMN-) Modulatoren sind Substanzen, die dazu fuhren, daß die Phagozytenaktivität (der einzelnen Zellen und/oder des Phagozyten-Gesamtsystems) moduliert, d.h. aktiviert oder supprimiert, wird. Die Phagozytenaktivität kann beispielsweise gemessen werden, indem die Erzeugung reaktiver Sauerstoff-Spezies (über Atmungskettenexplosion), wie Licht-emittierender Oxidantien (Chemilumineszenz) und/oder die fibrinauflösende, insbesondere antithrombotische, vorzugsweise selektiv (d.h. das plasmatische Gerinnungssystem nicht alterierend) thrombolytische Aktivität und/oder die chemotaktische Kapazität und/oder die Plasminaktivität dieser Zellen in vitro oder in vivo gemessen wird.

Erfindungsgemäß werden Phagozytenmodulatoren, insbesondere Phagozytenaktivatoren, zur antithrombotischen (antikoagulanten und/oder profibrinolytischen) und/oder antiatheroskierotischen Behandlung und/oder hämorrhagischen Behandlung und/oder Prophylaxe eingesetzt.

Phagozytensuppressoren werden erfindungsgemäß zur antihämorrhagischen Behandlung und/oder Prophylaxe phagozytenaktivierungsbedingter Hämostasestörungen eingesetzt.

Als Phagozytenaktivatoren eignen sich beispielsweise oxidierte Blut- oder Plasmaprodukte, d.h. oxidierte Rezeptoren (Reaktionspartner) für nichtradikalische Oxidantien. Diese Oxidations-Rezeptoren befinden sich beispielsweise im normalen Blut, nach Oxidation aktivieren (stimulieren) sie Phagozyten.

Beispiele für erfindungsgemäße phagozytenaktivierende Antithrombotika (d.h. Antikoagulantien und/oder Thrombolytika) und/oder Antiatherosklerotika sind oxidierte Blut- und/oder Plasmaprodukte und/oder oxidierte Oxidationsrezeptoren, insbesondere auch synthetische oxidierte Oxidationsrezeptoren, die den im Blut vorkommenden Oxidationsrezeptoren vergleichbar sind.

Beispielsweise kann ein 75 kg schwerer Patient mit 1 - 1000 ml Plasma, welches mit 0,1 - 100 mmol/l, vorzugsweise 1-40 mmol/l, besonders bevorzugt 2-15 mmol/1 eines Oxidans (wie beispielsweise NaOC1 oder ein nichtradikalisches Oxidans, wie Chloramin, vorzugsweise ein physiologisches Chloramin wie N-Chor-Taurin) voroxidiert wurde, antithrombotisch behandelt werden.

Oxidationsrezeptoren sind Moleküle mit für angeregten Sauerstoff empfindlichen Strukturen, beispielsweise Schwefelatome in Methionin oder Cyst(e)in oder C=C Gruppen. Auch sogenannte Redoxcycler, d.h. Substanzen, die reduktiv aktiviert werden und durch schrittweise Oxidation reaktive Sauerstoffspezies erzeugen können, welche mit Oxidationsrezeptoren zu Phagozytenaktivatoren reagieren, eignen sich zum erfindungsgemäßen antithrombotischen Verfahren durch Phagozyten-Aktivierung.

Beispiele für Redoxcycler sind Tetracyclin oder Chinonderivate.

Weitere Beispiele für Phagozytenaktivatoren sind
- Zellhormone, wie Granulocyte colony-stimulating factor (GM-CSF), das Peptidylmimetikum SB 247464, Gamma-Interferon, Interleukine (vorzugsweise Interleukin-6, Interleukin-8, Interleukin-10), Chemokine, Tumor necrosis factor (TNF), Thrombopoietin,
- mikrobielle Substanzen, wie Lipopolysaccharid, Liposom-muramyl-tripeptid-phosphalidylethanolamin, (opsonisiertes) Zymosan, Staphylococcus aureus Cowan I, Borrelia burgdorferi outer surface protein A (OspA), Beta-Glucan (von Saccharomyces cerevisiae), oder Galactoside-specific lectin (von Viscum album),
- immunologische und/oder chemotaktische Substanzen, wie Komplement (C) Spaltprodukt C3a, C5a, und/oder Lymphozyten- oder Monozytenprodukte
- Lipide/Lipidderivate, wie Leukotriene (insbesondere Leukotrien B4), Produkte der Reaktion der Phospholipase, beispielsweise der A₂-, C- oder D-Phospholipase mit Bestandteilen der Zellmembran, wie Arachidonsäure, 1,2 Diacylglycerol, Phosphatidylsäure, 1-Oleyl-2-acetyl-sn-glycerol, 1-Alkyl-2-acetyl-sn-glycero-3-phosphocholine (platelet activating factor), Diradylglycerol, 5-Oxo-eicosatetraenoic acid, Lipid-like leukocyte activator (LILA) oder oxidierte ungesättigte Fettsäuren und Fettsäurederivate,
- oder sonstige Phagozytenaktivatoren, wie Pyrithioxin, Aminoademantan, Antibiotika (insbesondere Staphylokokkenantibiotika, wie Fosfomycin ((1R,2S)-1,2-Epoxypropylphosphonsäure) oder Vancomycin), Dapson, Retinoide, Photonen (insbesondere des Licht-Wellenlängenbereichs 350-450 nm), angeregte Oxidantien und deren Freisetzer, Fluoride, ADP, Leukocyte Inhibitory Factor (LIF), Regulated upon activation of normal T cell expressed and secreted (RANTES), N-Formyl-methionyl-leucyl-phenylalanin, Eotaxin, W-7 (Calmodulin Antagonist), Lektine, wie Galectin-3 oder Galaptin, S100 Protein MRP- 14, aktivierte Gerinnungsproteine oder oxidiertes Albumin.

Beispiele für Phagozytensuppressoren sind Serinprotease / Serinprotease Inhibitor-Komplexe und/oder oxidierte Serinprotease Inhibitoren, insbesondere oxidiertes Antithrombin III, und/oder Serinprotease Inhibitor-Neoantigene.

Erfindungsgemäß ist die Verwendung von Phagozytenmodulatoren, insbesondere von Phagozytenaktivatoren, deren Derivate, Analoga, Mimetika (insbesondere Peptidmimetika), Vorstufen (beispielsweise Substanzen, die insbesondere in vivo zu Phagozytenmodulatoren umgewandelt werden) oder Antagonisten zur Prophylaxe und/oder Therapie thrombotischer, atherothrombotischer und/oder hämostaseologischer Erkrankungen. Phagozytenmodulatoren (vorzugsweise Phagozytenaktivatoren, insbesondere PMN-Aktivationen) eignen sich auch für Erkrankungen, bei denen eine Verbesserung der Abwehrfunktion der Phagozyten (z.B. bei infektiösen Erkrankungen, inbesondere Viruserkrankungen) klinisch indiziert ist.

Die Erfindung wird durch folgende Beispiele weiter erläutert und soll die Erfindung in keiner Weise einschränken.

### Beispiel 1:

### Phagozytenaktivatoren zur antithrombotischen (d.h. thrombolytischen und/oder antikoagulanten) Behandlung

In einem Kaninchen-Thrombolyse-Modell wurde eine Thrombose in der Vena jugularis gesetzt durch systemische Applikation eines aktivierten Prothrombinkomplexes und Stauung der Vena jugularis. Die Kaninchen wurden durch eine intramuskuläre Injektion einer Kombination aus Ketamin (80 mg/kg) und Xylazin (20 mg/kg) anästhesiert. Für Blutproben-Entnahmen wurde ein Katheter in die Arteria carotis plaziert. Dann wurden beide Jugularvenen über eine Länge von ca. 4 cm vorsichtig freipräpariert. 10 Einheiten/kg Factor Eight Inhibitor Bypassing Activity (FEIBA = aktiviertes pro-thrombotisches Prothrombin-Komplex-Konzentrat; Immuno AG) wurden in die Ohrrandvene injiziert. 1 min später wurden die Jugularvenen mit jeweils 3 mikrochirurgischen Gefäßklemmen über eine Länge von 2 cm abgeklemmt. Nach 15 minütiger Stase-Zeit wurden die distaten Gefäßklammern vollständig, die proximalen nur teilweise gelöst, um einen eventuell wieder einsetzenden Blutfluß zu gewährleisten und gleichzeitig das Risiko einer Embolie zu reduzieren. Die rechte Jugularvene wurde exzidiert und das Blut des Kaninchen dann mit 0,7 oder 35 µmol/kg Chloramin (N-Chlor-p-Toluensulfonamid (Chlorimin T^{R}), N-Chlor-Taurin oder Vancomycin (1 Mol Vancomycin = 2 Mol Chloramin)) in 17 ml physiol. NaC1 innerhalb 30 min gleichmäßig durch Verwendung einer Infusionspumpe oxidiert. 60 min nach Oxidansgabe wurde die linke Jugularvene exzidiert. Die Thromben wurden gewogen, in Formalin fixiert und histopathologisch (Hämatoxilin-Eosin- oder Chloracetat-Esterase (Phagozytenfärbung) gefärbt) untersucht. Plättchen-reiches Plasma (PRP) von thrombotischen Kaninchen, die mit 0, 7 oder 35 µmol/kg Chloramin behandelt wurden, wurde vor FEIBA-Injektion, 2 min nach FEIBA, 30 und 60 min nach Chloramin-Gabe auf Aggregabilität durch Zusatz von 1,25 oder 2,5 µmol/1 (Endkonz.) ADP zu 400 µl PRP untersucht (PAP-4, Biodata Corp., Horsham, USA). Falls für die Narkose der Kaninchen mehr Narkotikum erforderlich war, wurde 30 mg/kg Ketamin intramuskulär nachinjiziert, bis komplette Sedierung erreicht wurde. Die Tiere wurden nach Abschluß des Versuchs sofort durch eine intravenöse Überdosis Pentobarbital getötet. In einer modifizierten Versuchsversion wurde das nichtradikalische Oxidans vor Induktion der Thrombose verabreicht.

### Ergebnis:

Bei der durch Phagozytenaktivatoren (z.B. oxidierte Oxidationsrezeptoren des Blutes) induzierten Thrombolyse waren die globalen Gerinnungsteste aktivierte partielle Thromboplastinzeit (APTT), Prothrombinzeit (PT), Thrombinzeit (TT) und Thrombelastogramm (TEG) sowie Plättchenaggregometrie, Plasminogen-AktivatorInhibitor (PAI) Aktivität und D-Dimere nur geringfügig oder gar nicht verändert, d.h. die Thrombolyse ist selektiv. 15 min nach Injektion des Oxidans kam es zu einem vorübergehenden Absinken um 25 ± 13% an PMN und 40 ± 24% an Monozyten. Das Gewicht der Kontroll-Thrombi war 86 ±23 mg, die Gerinnsel der oxidativ behandelten Tiere wogen 2,8 ± 2.6 mg. Nach Phagozytenaktivierung stieg der Gehalt an polymorphkernigen Granulozyten im Thrombus mehr als 1000-fach: die Kontroll-Thrombi zeigten eine Granulozyten/Erythrozyten Ratio von etwa 1/2000, die oxidativ behandelten Gerinnsel eine von bis zu 10/1. Wird die Phagozyten-Aktivierung vor Induktion der Thrombose durchgeführt, so entsteht kein Thrombus, d.h. Phagozytenaktivatoren sind sowohl thrombolytisch als auch antikoagulant.

### Beispiel 2: Serinprotease/Serinprotease-Inhibitor Komplexe als Phagozytensuppressoren

20 µl heparinisiertes Plasma, zu welchem 0, 10 oder 100 IU/ml Rinder-Thrombin zugesetzt worden waren, oder 20 µl 200 IU/ml Human-Urokinase in Phosphatgepufferter physiol. NaCl oder 20µl PBS, welche 30 min. (37°C) mit 0 oder 50 Einheiten/ml Plasminogen Aktivator Inhibitor-2 inkubiert worden waren, wurden zu verdünntem Normal-Blut zugefügt und der Vollblut-Chemilumineszenz wie folgt gemessen: heparinisiertes (15 IU/ml) Blut von normalen Spendern wurde 10-fach mit Hanks Balanced Satt Solution (HBSS; Calcium enthaltend) verdünnt. 200 µl verdünntes Blut wurde auf eine Mikrotiterplatte pipettiert. 10 µl 2 mmol/l Luminol und 10 µl 1 µg/ml Phorbolmyristatacetat (Endkonz. 1,6 µmol/l) wurden zugesetzt und die Chemilumineszenz über 30 min. (37°C) verfolgt.

### Ergebnis:

Thrombin-(1 IU/ml oder 10 IU/ml) behandelte heparinisierte Plasmaproben, d.h. die dadurch generierten Thrombin-Antithrombin-Komplexe, erniedrigten die Chemiluminiszenz um 20 ± 4% (1 IU/ml Thrombin) und 98 ± 5% (10 IU/ml Thrombin). Urokinase-behandelte PAI-2 Proben sowie dieselbe Konzentration (5 Einheiten/ml Testkonzentration) an PAI-2 erniedrigten die Chemilumineszenz um mehr als 75%.

## Patentansprüche

1. Pharmazeutische Zusammensetzung enthaltend ein phagozytenmodulierendes Agens oder eine Vorstufe desselben zur Behandlung oder Prophylaxe von hämorrhagischen Erkrankungen.

2. Pharmazeutische Zusammensetzung enthaltend ein phagozytenmodulierendes Agens oder eine Vorstufe desselben zur Behandlung oder Prophylaxe von atherothrombotischen Erkrankungen.

3. Pharmazeutische Zusammensetzung enthaltend ein phagozytenmodulierendes Agens oder eine Vorstufe desselben zur Behandlung oder Prophylaxe von thrombotischen Erkrankungen, mit der Maßgabe, daß von einer Anregungsstrahlung unabhängige Singulett-Sauerstoff- und/oder Photonen-erzeugende Agenzien oder deren Vorstufen ausgeschlossen sind.

4. Pharmazeutische Zusammensetzung enthaltend ein phagozytenmodulierendes Agens oder eine Vorstufe desselben zur Behandlung oder Prophylaxe von infektiösen Erkrankungen, vorzugsweise Viruserkrankungen, mit der Maßgabe, daß von einer Anregungsstrahlung unabhängige Singulett-Sauerstoff- und/oder Photonen-erzeugende Agenzien oder deren Vorstufen ausgeschlossen sind.

5. Pharmazeutische Zusammensetzung nach einem der Ansprüche 1 bis 4, dadurch gekennzeichnet, daß das phagozytenmodulierende Agenz ein phagozytenstimulierendes Agens ist.

6. Pharmazeutische Zusammensetzung Anspruch 5, dadurch gekennzeichnet, daß das phagozytenstimulierende Agens ein oxidiertes Blut- und/oder Plasmaprodukt und/oder oxidierter Oxidationsrezeptor ist.

7. Pharmazeutische Zusammensetzung Anspruch 5, dadurch gekennzeichnet, daß das phagozytenmodulierende Agens ausgewählt wird aus der Gruppe bestehend aus Zeilhormonen, mikrobiellen Substanzen, immunologischen und/oder chemotaktischen Substanzen, Lipiden, Lipidderivaten, Pyrithioxin, Amino-ademantan, Antibiotika, insbesondere Staphylokokkenantibiotika, Dapson, Retinoiden, Photonen, angeregten Oxidantien und deren Freisetzern, Fluoriden, ADP, Leukocyte Inhibitory Factor (LIF), Regulated upon activation of normal T cell expressed and secreted (RANTES), N-Formyl-methionyl-leucyl-phenylalanin, Eotaxin, W-7 (Calmodulin Antagonist), Lektinen, insbesondere Galectin-3 oder Galaptin, S100 Protein MRP-14, aktivierten Gerinnungsproteinen und oxidiertem Albumin.

8. Pharmazeutische Zusammensetzung Anspruch 7, dadurch gekennzeichnet, daß das Zellhormon ausgewählt wird aus der Gruppe bestehend aus Granulocyte colony-stimulating factor (GM-CSF), dem Peptidyltmimetikum SB 247464, Gamma-Interferon, Interleukinen, insbesondere Interleukin-6, Interleukin-8 und Interleukin-10, Chemokinen, Tumor necrosis factor (TNF) und Thrombopoietin; daß die mikrobiellen Substanzen ausgewählt werden aus der Gruppe bestehend aus Lipopolysaccharid, Liposom-muramyl-tripeptidphophalidylethanolamin, (opsonisiertes) Zymosan, Staphylococcus aureus Cowan I, Borrelia burgdorferi outer surface protein A (OspA), Beta-Glucan, insbesondere von Saccharomyces cerevisiae, und Galactoside-specific lectin, insbesondere von Viscum album; daß die immunologischen und/oder chemotaktischen Substanzen ausgewählt werden aus der Gruppe bestehend aus Komplement (C), Spaltprodukt C3a, C5a, und Lymphozyten- oder Monozytenprodukten; daß die Lipide und Lipidderivate ausgewählt werden aus der Gruppe bestehend aus Leukotrienen, insbesondere Leukotrien B4, Produkte der Reaktion der Phospholipase mit Bestandteilen der Zellmembran, insbesondere Arachidonsäure, 1,2 Diacylglycerol, Phosphatidylsäure, 1-Oleyl-2-acetyl-sn-glycerol, 1-Alkyl-2-acetyl-sn-glycero-3-phosphocholine (platelet activating factor), Diradylglycerol, 5-Oxo-eicosatetraenoic acid, Lipid-like leukocyte activator (LILA) oder oxidierten ungesättigten Fettsäuren und Fettsäurederivaten.

9. Pharmazeutische Zusammensetzung nach einem der Ansprüche 1 bis 4, dadurch gekennzeichnet, daß das phagozytenmodulierende Agenz ein phagozytensupprimierendes Agens ist.

10. Pharmazeutische Zusammensetzung nach Anspruch 9, dadurch gekennzeichnet, daß das phagozyten-supprimierende Agens ausgewahlt wird aus der Gruppe bestehend aus Serinprotease / Serinprotease Inhibitor-Komplexen und/oder oxidierten Serinprotease Inhibitoren, insbesondere oxidiertem Antithrombin III, und/oder Serinprotease Inhibitor-Neoantigenen.
